(19) **Europäisches Patentamt / European Patent Office / Office européen des brevets**

(11) **EP 4 299 113 A1**

(12) # EUROPEAN PATENT APPLICATION

(43) Date of publication:
**03.01.2024 Bulletin 2024/01**

(21) Application number: **22181596.2**

(22) Date of filing: **28.06.2022**

(51) International Patent Classification (IPC):
***A61N 5/10*** *(2006.01)*

(52) Cooperative Patent Classification (CPC):
**A61N 5/1031;** A61N 5/1001; A61N 5/1071

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **RaySearch Laboratories AB**
**104 30 Stockholm (SE)**

(72) Inventors:
• **NIESSEN, Tom**
**104 30 STOCKHOLM (SE)**
• **ENGWALL, Erik**
**129 44 Hägersten (SE)**

(54) **METHOD, COMPUTER PROGRAM PRODUCT AND COMPUTER SYSTEM FOR DOSE CALCULATION**

(57) A dose calculation method for brachytherapy treatment, is disclosed, in which radiation is provided by one or more radiation sources from at least a first and a second dwell position within a patient. The method comprises performing a first dose calculation for the first dwell position according to a first dose calculation algorithm and a second dose calculation for the second dwell position according to a second dose calculation algorithm, and using the sum of the first dose calculation and the second dose calculations as the total calculated dose. Different dose engines with different properties can be combined to provide time-efficient calculations with satisfactory accuracy.

FIG. 2

EP 4 299 113 A1

## Description

### Technical Field

The present invention relates to radiation therapy treatment and more specifically to brachytherapy.

### Background

Unlike external beam radiation therapy, in which radiation is delivered to a patient in the form of a beam from the outside, brachytherapy involves inserting a radiation source into the patient inside or next to the target volume. The radiation source may be in the form of a seed, a ribbon or capsule. Brachytherapy involves a number of advantages, including that the radiation source can be placed in close proximity to the target, and that it remains in place, relative to the target, even as the whole patient moves or when there are internal movements within the patient. It also allows for high dose rates, which enables treatments in just a few fractions.

In some forms of brachytherapy, the radiation source is placed inside and moved through the patient by utilizing a natural cavity or interstitial channel, typically inserted with one or more catheters. The radiation source is moved through the channel and stops at predefined positions along the trajectory, known as dwell positions. The time spent in each dwell position, the dwell time, is determined individually for each position and may typically be on the order of 1 - 2 seconds.

As with any type of radiation therapy, it is important to calculate the resulting dose to different parts of the patient. Because the radiation source radiates in all directions from every dwell position inside the patient this requires a lot of calculation. In general, known algorithms for such calculations are either based on simplified assumptions which make the calculations fast but not always accurate, or based on dose engine calculations which are more accurate but time consuming.

One way of calculating the dose involves an analytical algorithm which uses the cylindrical symmetry of the dose distributed by the source. This algorithm is based on the assumption that the surrounding tissue is water equivalent and homogeneous. This simplification means that the algorithm is fast but also that it is not necessarily accurate, in particular with heterogeneous tissue or tissue having material properties that is very different from water. Relevant material properties include density, effective electron density, photo-electric effect, and Compton scattering.

Another way of calculating the dose involves a Monte Carlo dose engine. Such dose calculations may be performed with great accuracy but are presently so time-consuming that they might not be feasible for clinical use, especially when it comes to optimizing the treatment plan.

### Summary

It is an object of the present disclosure to provide a dose calculation method for brachytherapy which is both accurate and fast compared to the prior art methods.

The present disclosure relates to a dose calculation method for brachytherapy treatment, in which radiation is provided by one or more radiation sources from at least a first and a second dwell position within a patient. The method comprises performing a first dose calculation for the first dwell position according to a first dose calculation algorithm and a second dose calculation for the second dwell position according to a second dose calculation algorithm, and using the sum of the first dose calculation and the second dose calculations as the total calculated dose.

Such a method enables the optimal combination of dose calculation methods with regard to time and accuracy, in view of patient anatomy. The method identifies positions in which a more complex algorithm is needed for an accurate result. Algorithms that are fast but less accurate may be used where such an algorithm will return a satisfactory result. Thus, the method will return accurate values in complex areas and save time by using a faster method in areas where less accuracy is needed, that is, areas where the surrounding tissue has a density that is close to water and shows little variation. Therefore, a satisfactory result can be achieved with reasonable calculation times.

The first dose calculation algorithm may be an analytical algorithm assuming a homogeneous density of the tissue surrounding the first dwell position, for example, the density of water. Such algorithms include ray-tracing and TG43, which will be fast but not always very accurate.

The second dose calculation algorithm is preferably arranged to take heterogeneities into account. Such algorithms will be slower but will yield a more accurate result. Examples of such algorithms include MC dose engine and Boltzmann equation solver.

The dose calculation algorithm for each dwell position is preferably selected based on a heterogeneity index for the dwell position, said heterogeneity index indicating the variation of tissue properties in the volume surrounding the dwell position. The faster algorithm is selected for areas with low heterogeneity and the slower more accurate algorithm is used for areas where heterogeneity is high.

The heterogeneity index for each dwell position may be calculated based on ray tracing in two or more directions near the dwell position and determining the tissue properties for intersected voxels for each ray tracing operation.

Alternatively, the heterogeneity index for each dwell position is calculated based on defining a sub-volume around each dwell position, each sub-volume including a plurality of voxels and determining the tissue properties in at least some of the plurality of voxels. The variation in tissue properties will indicate the degree of heterogeneity so that large variations will result in a high heterogeneity index whereas small or no variation will result in a low heterogeneity index.

Instead of, or in addition to the heterogeneity index discussed above, the dose calculation method for each dwell position may be selected based on a comparison with a homogeneous reference material, such as water. This would enable the selection of different dose calculation methods for different homogeneous tissues as well. Typically, tissue having a different density from the reference material would indicate that a more accurate dose calculate method should be used, even if the tissue has a low heterogeneity index.

More than two dose calculation algorithms can be used. For example, a fast, an intermediate and a slow but accurate algorithm can be used for the less critical, somewhat critical and very critical areas, respectively. Typically, the highly heterogeneous areas will be the most critical ones whereas highly homogenous areas can be handled using a fast algorithm.

The dose calculation method according to the invention may be used in any suitable context but is particularly useful for optimization. Accordingly, the disclosure also relates to a brachytherapy optimization method for optimizing a set of dwell times for a corresponding set of dwell positions. The brachytherapy optimization method includes calculating the total dose using a method according to any of the embodiments above and using the calculated total dose in the optimization procedure.

The disclosure also relates to a computer program product arranged to perform dose calculations according to a first and a second dose calculating algorithm, said computer program product comprising computer-readable code means which, when executed in a computing device will cause the computing device to perform the method according to any of the embodiments above. As is common in the art, the code means may be stored on a non-transitory memory. The disclosure also relates to a computer comprising a processor and a program memory, said program memory having stored therein a such a computer program product in such a way that it can be executed by the processor.

## Brief description of drawings

The invention will be described in more detail in the following, by way of examples and with reference to the appended drawings.

Figure 1 illustrates a portion of a patient undergoing brachytherapy.
Figure 2 is a flow chart of a method according to an embodiment of the invention.
Figures 3 and 4 illustrate possible ways of calculating the index value.
Figure 5 is a more detailed flow chart of a part of the flow chart in Figure 2.

## Detailed description of embodiments

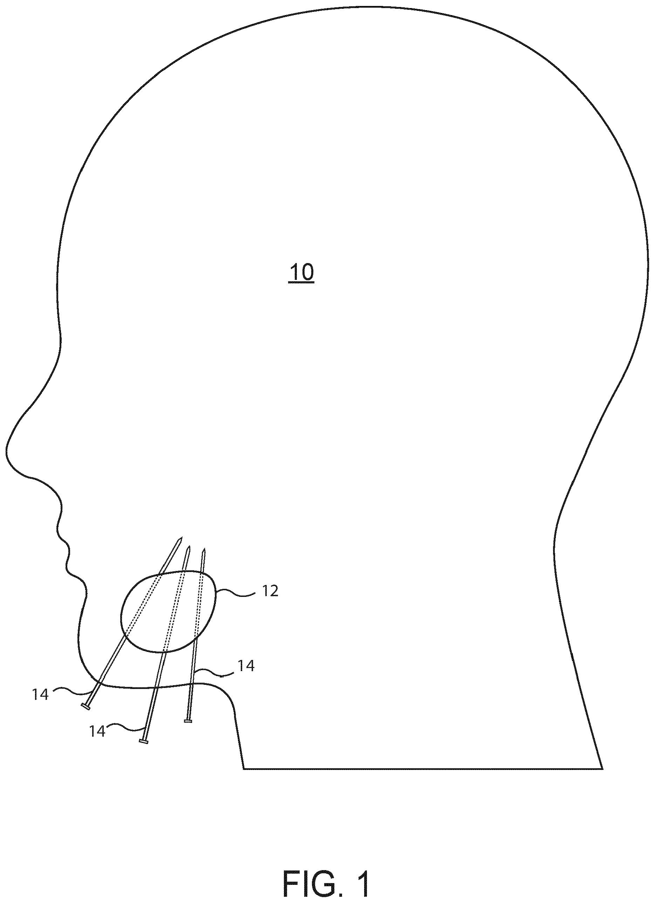

Figure 1 illustrates a head of a patient 10 undergoing brachytherapy, seen in profile , including a treatment site around a target 12 that is to be exposed to radiation. Three channels 14 is provided, through which a radiation source is to be passed in a manner known in the art. As will be understood, there will typically be several channels, each with a number of dwell positions. For clarity only three channels are shown here. The treatment plan includes a number of dwell positions within each channel 14, where the radiation source will stop temporarily, and a dwell time for each dwell position indicating how long the radiation source will stop in that position. The total dose will be the sum of the doses delivered from all of the dwell positions. Shields (not shown) can be applied to stop radiation in certain directions to protect organs at risk. As will be understood, the geometry of the treatment site is close to homogenous in some areas and more heterogeneous in other areas because of air cavities, bone, teeth and possibly other materials. This will affect the dose delivered in different directions outwards from the radiation source in its different positions. How to implement this is known in the art, including determining dwell positions and the nature of the radiation source. The radiation source geometry also affects the dose in different directions.

In some embodiments, the fast but less exact dose calculation algorithm is the commonly used analytical TG43 dose algorithm. Examples of dose calculation algorithms that are exact but slower, include dose engine algorithms, such as the Monte Carlo based algorithm.

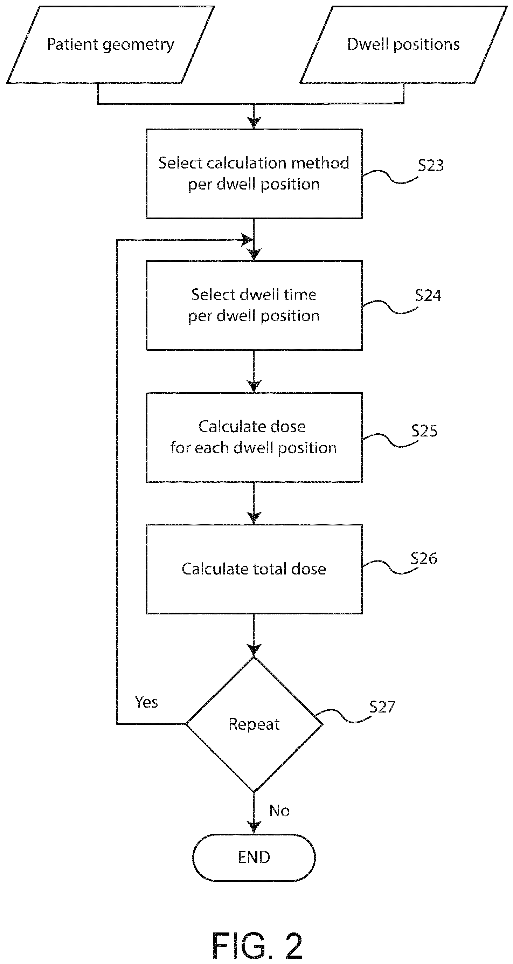

Figure 2 is a flow chart of a method according to an embodiment of the invention. The method is performed after the target and any organs at risk have been identified S21, as well as a number of dwell positions S22 within the treatment area. This constitutes input data to the method.

The total dose resulting from all dwell positions is needed during treatment plan optimization, but is also needed when computing the final dose of the resulting treatment plan. The final dose is used for evaluating the prescription and clinical goals and approve the planned treatment for delivery. The accuracy requirement is higher for the final dose than for the dose used during optimization. According to the present disclosure two or more different dose calculation algorithms having different properties are used. For each dwell position the most suitable dose calculation algorithm is selected in step S23. The selection of dose calculation algorithm for each dwell position can be based on any suitable set of criteria. In some embodiments, the selection is made based on an index value that is indicative of either the difference in material properties such as density between water and the tissue surrounding the dwell position, or the variation in material properties in the tissue surrounding the dwell position, or a combination of the two. How to determine the index value will be discussed in more detail below.

In step S24, the dose from the source at each dwell position is calculated, using the algorithm selected for that dwell position in step S23. In step 25 the dwell time for each dwell position is selected. In step S26, the total resulting dose is calculated as the weighted sum of all the doses calculated in step S24 weighted by the respective dwell times in the dwell positions obtained in step 25. The method may include calculating dose contributions from dwell positions belonging to a number of channels or cavities within the patient.

The method according to Figure 2 may be used as part of an optimization procedure, in which the dwell times are used as the optimization variables in a dose-based optimization, and the dwell times are changed based on fulfillment of user-specified optimization functions equated over the total resulting dose. This is indicated by an optional step S27 which will allow the method to be repeated from step S25 until the optimization is terminated, which can occur, for example, when a predefined number of iterations is reached or when the optimization functions have been fulfilled, for example when a suitable total dose has been achieved.

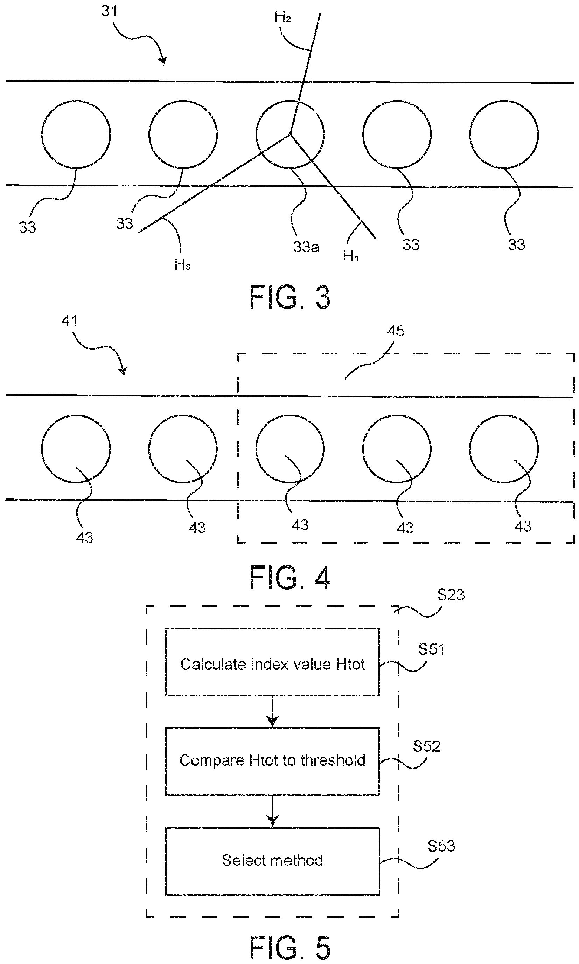

Figure 3 illustrates a first method of calculating the index value referred to above. Reference numeral 31 indicates a channel through which a radiation source is to be passed. Within the channel a number of dwell points 33 are defined. One dwell point 33a is selected for illustration purposes. For this selected dwell point 33a, a number of directions (three are shown in Figure 3) are defined or sampled, and ray tracing is performed in these directions. Ray tracing involves accumulating a term for each intersected voxel i in the ray trace dependent on the relevant tissue properties, indicated here as p. Suitable tissue properties to consider include tissue density, effective electron density, cross sections for photoelectric effect or Compton scattering. The resulting terms are labeled $H_1$, $H_2$, $H_3$ in Fig. 3. Since the dose from the source in each dwell position falls off with increasing distance, the term could preferably fall off with increasing distance from the dwell position to account for that material variations close to the source will have a larger impact on the dose distribution than those further away. The index value for the selected dwell point is calculated based on the results of these three ray traces as the sum, possibly weighted sum over all ray-traces j. Since dwell positions are normally positioned close to each other, the index value can also be calculated for a group of neighboring dwell positions rather than for each dwell position individually.

The contribution to the index value from each ray trace in the example in Fig. 3 may be expressed as Eq. 1:

$$H = \sum_i \frac{|\rho_i - \rho_{water}|}{r_i^2} \qquad (1)$$

That is, the sum over all voxels i of the difference between the tissue density of the voxel and the density of water, divided by the square of the distance r between the voxel i and the center of the dwell point. Instead of the difference in tissue density, p could represent some other material property, or combination of two or more properties. The numerator could also be written as the relative difference in the material property between tissue and water. Additionally, more than one material property can be considered, in which case $\rho_i$- $\rho_{water}$ would be a sum over the relative differences of a set of material properties.

The total index value Htot in this case may be expressed as Eq. 2:

$$H_{tot} = \sum_j H_j \qquad (2)$$

That is, the sum of all index value contributions calculated according to Eq. 1 for all selected directions j for ray tracing.

Alternatively, or in addition to the comparison with water, a comparison between tissue properties of different voxels within the relevant volume can be used as an indicator of heterogeneity. In that case, Eq.1 would be replaced by Eq. 1a:

$$H = \sum_i \frac{|\rho_i - \rho_{mean}|}{r_i^2} \qquad (1a)$$

where $\rho_{mean}$ expresses the variance of the material property or properties, that is, the mean value of all ray-traced voxels.

If the tissue surrounding the dwell position is relatively homogenous, it may still be desirable to select a more accurate dose calculation method. Therefore, the selection of dose engine could also be based on the difference in tissue density between the tissue surrounding the dwell position and a reference density value, where a difference exceeding a set threshold would indicate that a more accurate dose calculation method should be used. A suitable reference density value would often be the density of water.

Figure 4 illustrates a second way of determining the index value H. As in Fig. 3, a channel 41 is shown, including a number of dwell positions 43. In this case a sub-volume 45 is defined around a dwell position. The relevant tissue property or properties are determined for all voxels within the sub-volume and the index value is calculated by accumulating the tissue property values for these voxels. The procedure may be made more efficient by considering a number of adjacent dwell positions together, that is, define one sub-volume covering a plurality of dwell positions, and/or by caching part of the heterogeneity index per sub-volume. In this case, the total index value Htot is given by Eq. 3:

$$H_{tot} = \sum_i \frac{|\rho_i - \rho_{water}|}{r_i^2} \qquad (3)$$

That is, the index value Htot is given as the sum over all voxels in the sub-volume 45 of the difference between the tissue density of the voxel and the density of water, divided by the square of the distance r between the voxel i and the center of the dwell point. As for Eq. (1), some other material property, relative differences or differences for a sequence of material properties could be considered instead of the density. Also, another homogeneous density could be used instead of the density of water. As for the method involving ray tracing, a comparison between tissue properties of different voxels within the relevant volume can be used as an indicator of heterogeneity instead of, or in addition to, the comparison with water.

The selection in step S23 can therefore be broken down into substeps as illustrated in Fig. 5: In a first step S51, the index value Htot for a dwell position or a group of dwell positions is calculated, either using ray tracing and equations 1 and 2, or using sub-volumes and equation 3. In step S52 the index value Htot is compared to a threshold value. If the index value $H_{tot}$ is lower than the threshold, this indicates tissue properties that are similar to those of water and/or relatively homogenous, meaning that a calculation algorithm that is fast may be used for the dwell position or positions concerned even though it is less exact. If the index value Htot is higher than the threshold, this indicates tissue properties that are different from those of water and/or non-homogenous, meaning that a calculation algorithm that is more exact but more time consuming should be used for the dwell position or positions concerned. As will be understood, there may be several different algorithms and thresholds for each of them so that below a lowermost threshold a very fast algorithm may be used, between the lowermost threshold and a higher threshold an intermediate algorithm may be used and above the higher threshold a slower but very accurate algorithm can be used.

In the simplest case, two calculation algorithms are used, one that is accurate but slow for where accuracy is needed, and one that is faster and less accurate for voxels that are less critical. For the accurate calculation algorithm, a dose engine such as a Monte Carlo dose engine or Boltzmann equation solvers could be used. For the fast, but less accurate calculation algorithm, the analytical TG43 dose engine is, as previously mentioned, most commonly used. Algorithms using accurate and less accurate dose engines can be combined depending on their properties and the desired dose calculation speed. Examples of dose calculation algorithms that may be used include:

- Monte Carlo (MC). This algorithm is very accurate but is typically slower than other algorithms. Time scales with the size of the volume in which dose is to be calculated and with the number of approximations made inside the MC engine. Time scales inversely with desired statistical uncertainty.
- Boltzmann equation solver. Speed and accuracy are comparable to Monte Carlo.
- Collapsed cone (CC). Intermediately fast. Can take tissue heterogeneities into account, but includes approximations that make the algorithm less accurate under certain conditions, such as high degrees of heterogeneities, high energies or large treatment volumes.
- Pencil beam. Fast, but less accurate under inhomogeneous conditions.
- SVD. Faster than pencil beam and CC. Comparable to pencil beam in terms of accuracy.
- Ray-tracing. Very fast, but accuracy is limited under inhomogeneous conditions.
- TG43, which is most commonly used at present. This algorithm is very fast, but tends to be inaccurate under inhomogeneous or water-dissimilar conditions.

For each of the listed algorithms different settings can be used that will affect the performance and the accuracy. The combination of different algorithms as described above, could also include using an algorithm performed by the same dose engine but with different settings.

**Claims**

1. A dose calculation method for brachytherapy treatment, in which radiation is provided by one or more radiation sources from at least a first and a second dwell position within a patient, the method comprising performing a first dose calculation for the first dwell position according to a first dose calculation algorithm and a second dose calculation for the second dwell position according to a second dose calculation algorithm, and using the sum of the first dose calculation and the second dose calculations as the total calculated dose.

2. A dose calculation method according to claim 1, wherein the first dose calculation algorithm is an analytical method assuming a homogeneous density of the tissue surrounding the first dwell position, for example, the density of water.

3. A dose calculation method according to claim 1 or 2, wherein the second dose calculation algorithm is arranged to take into account heterogeneities in tissue surrounding the second dwell position.

4. A method according to any one of the preceding claims, wherein the dose calculation method for each dwell position is selected based on a heterogeneity index for that dwell position, said heterogeneity index indicating the variation of tissue properties in the volume surrounding the dwell position.

5. A method according to claim 4, wherein the heterogeneity index for each dwell position is calculated based on ray tracing in two or more directions near that dwell position and determining the tissue properties for intersected voxels for each ray tracing operation.

6. A method according to claim 4, wherein the heterogeneity index for each dwell position is calculated based on defining a sub-volume around each dwell position, each sub-volume including a plurality of voxels and determining the tissue properties in at least some of the plurality of voxels.

7. A method according to any one of the preceding claims, wherein the dose calculation method for each dwell position is selected based on a comparison with a homogeneous reference material.

8. A method according to any one of the preceding claims, further comprising performing a third dose calculation for a third dwell position according to a third dose calculation algorithm, and using the sum of the first, second and third dose calculations as the total calculated dose.

9. A brachytherapy optimization method for optimizing a set of dwell times for a corresponding set of dwell positions, said optimization method includes calculating the total dose using a method according to any one of the preceding claims and using the calculated total dose in the optimization procedure.

10. A computer program product arranged to perform dose calculations according to a first and a second dose calculating algorithm, said computer program product comprising computer-readable code means which, when executed in a computing device will cause the computing device to perform the method according to any one of the preceding claims.

11. A computer program product comprising a non-transitory storage medium having stored thereon a computer program product according to claim 10.

12. A computer comprising a processor and a program memory, said program memory having stored therein a computer program product according to claim 11, in such a way that it can be executed by the processor.

FIG. 1

```
┌─────────────────────┐         ┌─────────────────────┐
│   Patient geometry  /         │   Dwell positions   /
└─────────────────────┘         └─────────────────────┘
```

Patient geometry

Dwell positions

Select calculation method
per dwell position — S23

Select dwell time
per dwell position — S24

Calculate dose
for each dwell position — S25

Calculate total dose — S26

Repeat — S27

Yes

No

END

## FIG. 2

31

$H_2$

33

33

$H_3$

33a

$H_1$

33

33

**FIG. 3**

41

45

43

43

43

43

43

**FIG. 4**

S23

| Calculate index value Htot | S51 |
| Compare Htot to threshold | S52 |
| Select method | S53 |

**FIG. 5**

Europäisches
Patentamt

European
Patent Office

Office européen
des brevets

## EUROPEAN SEARCH REPORT

Application Number

EP 22 18 1596

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/063110 A1 (FAILLA GREGORY ALEXANDER [US] ET AL) 5 March 2009 (2009-03-05) | 1-3,7-12 | INV. A61N5/10 |
| A | * the whole document * | 4-6 | |
| X | AHNESJÖ ANDERS ET AL: "Collapsed cone dose calculations for heterogeneous tissues in brachytherapy using primary and scatter separation source data", COMPUTER METHODS AND PROGRAMS IN BIOMEDICINE, ELSEVIER, AMSTERDAM, NL, vol. 139, 24 October 2016 (2016-10-24), pages 17-29, XP029913445, ISSN: 0169-2607, DOI: 10.1016/J.CMPB.2016.10.022 * abstract * * page 18, column 2 - page 23, column 1 * | 1-3,8-12 | |
| X | EP 2 431 074 A1 (UNIV LOUVAIN [BE]; ATOMIC ENERGY COMMISSION OF SYRIA AECS [SY]) 21 March 2012 (2012-03-21) * abstract; claim 1 * | 1,10-12 | TECHNICAL FIELDS SEARCHED (IPC) A61N |
| A | MAO XIMENG ET AL: "RapidBrachyDL: Rapid Radiation Dose Calculations in Brachytherapy Via Deep Learning", INTERNATIONAL JOURNAL OF RADIATION: ONCOLOGY BIOLOGY PHYSICS, PERGAMON PRESS, USA, vol. 108, no. 3, 12 May 2020 (2020-05-12), pages 802-812, XP086262804, ISSN: 0360-3016, DOI: 10.1016/J.IJROBP.2020.04.045 [retrieved on 2020-05-12] * the whole document * | 1-12 | |

-/--

The present search report has been drawn up for all claims

| Place of search | Date of completion of the search | Examiner |
|---|---|---|
| Munich | 25 November 2022 | Beck, Ewa |

EPO FORM 1503 03.82 (P04C01)

page 1 of 2

Europäisches
Patentamt

European
Patent Office

Office européen
des brevets

# EUROPEAN SEARCH REPORT

**Application Number**

**EP 22 18 1596**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHIBANI OMAR ET AL: "MCPI : A sub-minute Monte Carlo dose calculation engine for prostate implants", MEDICAL PHYSICS, AIP, MELVILLE, NY, US, vol. 32, no. 12, 17 November 2005 (2005-11-17), pages 3688-3698, XP012075232, ISSN: 0094-2405, DOI: 10.1118/1.2126822 * the whole document * | 1-12 | |
| A | CN 113 599 728 A (UNIV BEIHANG) 5 November 2021 (2021-11-05) * the whole document * | 1-12 | |

**TECHNICAL FIELDS SEARCHED (IPC)**

The present search report has been drawn up for all claims

| Place of search | Date of completion of the search | Examiner |
|---|---|---|
| Munich | 25 November 2022 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 22 18 1596

This annex lists the patent family members relating to the patent documents cited in the above-mentioned European search report.
The members are as contained in the European Patent Office EDP file on
The European Patent Office is in no way liable for these particulars which are merely given for the purpose of information.

25-11-2022

| Patent document cited in search report | | Publication date | Patent family member(s) | | Publication date |
|---|---|---|---|---|---|
| US 2009063110 | A1 | 05-03-2009 | NONE | | |
| EP 2431074 | A1 | 21-03-2012 | EP | 2431074 A1 | 21-03-2012 |
| | | | WO | 2012038458 A1 | 29-03-2012 |
| CN 113599728 | A | 05-11-2021 | NONE | | |

For more details about this annex : see Official Journal of the European Patent Office, No. 12/82